# EUROPÄISCHE PATENTSCHRIFT

(11) **EP 1 187 799 B1**
(45) Veröffentlichungstag und Bekanntmachung des Hinweises auf die Patenterteilung: **23.07.2003**
(21) Anmeldenummer: 00949207.5
(22) Anmeldetag: 21.06.2000
(51) Int. Cl.: C07C 41/03, C07C 43/23

(54) **VERFAHREN ZUR HERSTELLUNG VON BISPHENOLALKOXYLATEN**
METHOD FOR PRODUCING BISPHENOL ALCOXYLATES
PROCEDE DE PREPARATION D'ALCOXYLATES DE BISPHENOL

(30) Priorität: 22.06.1999 DE 19928549; 10.02.2000 DE 10005792
(43) Veröffentlichungstag der Anmeldung: 20.03.2002
(73) Patentinhaber: BASF AKTIENGESELLSCHAFT, 67056 Ludwigshafen (DE)
(72) Erfinder: KOSER, Stefan, D-67061 Ludwigshafen (DE); MUNDINGER, Klaus, D-67117 Limburgerhof (DE); KASEL, Wolfgang, D-69226 Nussloch (DE); KINGMA, Arend, Jouke, D-67069 Ludwigshafen (DE); DOCKNER, Toni, D-67149 Meckenheim (DE)
(74) Vertreter: Kinzebach, Werner, Dr.
(86) Internationale Anmeldenummer: EP0005753
(87) Internationale Veröffentlichungsnummer: WO00078698

(56) Entgegenhaltungen:
- EP-A- 0 466 319
- US-A- 4 846 996
- CHEMICAL ABSTRACTS, vol. 94, no. 8, 23. Februar 1981 (1981-02-23) Columbus, Ohio, US; abstract no. 48110d, Z.-C. SHENG: "Study on unsaturated polyester resins of the bisphenol A type. Part I. Syntheses of D-33 monomer and 323 resin" Seite 25; Spalte 2; XP002150879 in der Anmeldung erwähnt & SHANG-HAI HUA KUNG HSUEH PAO, Nr. 1, 1980,
- DATABASE WPI Section Ch, Week 8603 Derwent Publications Ltd., London, GB; Class E14, AN 1986-018517 XP002150880 & JP 60 243036 A (TAKEMOTO OIL & FAT CO) in der Anmeldung erwähnt

## Beschreibung

Die Erfindung betrifft ein Verfahren zur Herstellung von Bisphenolalkoxylaten, insbesondere Bisphenol A-Alkoxylaten. Außerdem betrifft die Erfindung die Verwendung der dazu eingesetzten Katalysatoren.

Bisphenolalkoxylate werden für die verschiedensten Anwendungen, beispielsweise für die Synthese von Polyesterharzen (JP 59012-934-A) und Polyurethanen (JP 59197-417-A) eingesetzt.

Bisphenol-Alkoxylate, insbesondere Bisphenol A-Alkoxylate, werden - wie dem Fachmann bekannt ist - ausgehend von Bisphenol A durch Umsetzung mit einem Alkoxid, z.B. Ethylenoxid, Propylenoxid oder Butylenoxid (JP 60243-036-A), in Anwesenheit eines Katalysators hergestellt. Als Katalysatoren werden vor allem Alkalimetallhydroxide wie Natriumhydroxid, Kaliumhydroxid oder tertiäre Amine eingesetzt (siehe die obigen JP-Publikationen sowie Shanghai Inst. Chem. Technol., Shanghai in Chemical Abstracts 94:48110).

Die Alkoxylierungsreaktion kann zu unterschiedlichen Additionsprodukten führen. Die bekannten Katalysatoren etwa führen zu einem Produkt, bei dem die Additionsprodukte eine relativ breite Molmassenverteilung aufweisen.

Wenn eine hohe Reaktionsselektivität gewünscht wird, werden zum Teil Lösungsmittel eingesetzt, um die Selektivität der Alkoxidaddition an Bisphenol A in der gewünschten Richtung zu beeinflussen (US-A 4,846,996). Diese Maßnahme führt allerdings zu einer Verringerung der Raum-Zeit-Ausbeute, da das eingesetzte Lösungsmittel nach Beendigung der Reaktion wieder entfernt werden muß.

Eine andere Lösungsmöglichkeit besteht darin, die Reaktion eines Phenols mit einem Alkylenoxid gegebenenfalls in Gegenwart eines Lösungsmittels unter Verwendung von Phosphoniumhalogeniden als Katalysatoren ablaufen zu lassen (JP AS 654/75; DE-A 2 157 455).

Eine weitere Maßnahme zur Erreichung eines Bisphenol A-Additionsproduktes mit einer engen Molmassenverteilung ist die Produktkristallisation. Dieses Vorgehen verringert aber ebenfalls die Raum-Zeit-Ausbeute und führt zusätzlich zu sinkenden Ausbeuten.

Sheng Zhicong et al. [Shang-hai Hua Kung Hsueh Yuan Hsueh Pao 1980, 48 (1), 92] beschreiben Untersuchungen zur Umsetzung von Propylenoxid mit Bisphenol A. Es wird angegeben, dass der bevorzugte Katalysator NaOH ist und dass die Katalysatormenge verringert werden kann, wenn eine Lewis-Base, wie Triethylamin oder Triphenylphosphin, mitverwendet wird (d.h. die Lewis-Base wird als Cokatalysator eingesetzt). Weitere Angaben, die den Fachmann in die Lage versetzen, die Umsetzung durchzuführen, sind in dieser Publikation jedoch nicht enthalten. Außerdem ergibt sich der unerwünschte Effekt, daß der Katalysator gegen Ende der Reaktion an Aktivität verliert und als Folge davon der Bisphenol A-Monopropoxylatgehalt erhöht ist.

Auch bei Verwendung anderer Alkoxide ist daher geringe Selektivität zu erwarten. Dies gilt vor allem für Ethylenoxid, da Ethylenoxid im Vergleich zu Propylenoxid wesentlich reaktiver ist. Der Fachmann würde daher die Bildung von mehrfach ethoxylierten Produkten und damit eine breitere Molmassenverteilung erwarten.

Aufgabe der Erfindung ist es daher ein Verfahren zur Herstellung von Bisphenolalkoxylaten, insbesondere Bisphenol A-Alkoxylaten, bereitzustellen, das mit hoher Selektivität dialkoxylierte Produkte ergibt, wobei insbesondere die ungesteuerte Mehrfachalkoxylierung vermieden werden soll.

Erfindungsgemäß wird diese Aufgabe durch ein Verfahren zur Herstellung von Bisphenolalkoxylaten gelöst, wobei man mindestens ein Bisphenol mit einem Alkylenoxid in Anwesenheit eines Phosphinkatalysators zur Reaktion bringt.

In einer bevorzugten Ausführungsform wird bei dem erfindungsgemäßen Verfahren ohne Lösungsmittel gearbeitet, d.h. es wird zunächst eine Mischung, insbesondere eine Schmelze aus dem Bisphenol und dem Phosphinkatalysator bereitgestellt, anschließend erfolgt die Reaktion mit dem Alkylenoxid. Die Umsetzung kann aber auch in Anwesenheit eines inerten Lösungsmittels erfolgen. Brauchbare Lösungsmittel sind z.B. Kohlenwasserstoffe, wie Toluol oder Xylol, Ketone, wie Methylethylketon oder Diethylketon.

Bevorzugte Alkylenoxide sind C₂-C₄-Alkylenoxide, insbesondere Ethylenoxid, Propylenoxid und 1,2-Butylenoxid und Gemische davon, wobei Ethylenoxid besonders bevorzugt ist. Auch Styroloxid ist geeignet.

Bei dem erfindungsgemäßen Verfahren werden als Katalysatoren Stoffe eingesetzt, die zur Klasse der substituierten Phosphine gehören. Diese sind bevorzugt ausgewählt aus der Gruppe der substituierten Phosphine der nachfolgenden Formel (I): 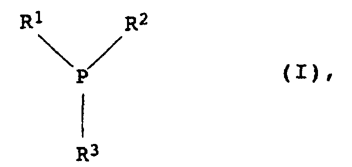 worin R¹, R² und R³ Arylreste gemäß der nachfolgenden Formel (II) sind, 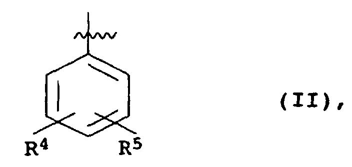 worin R⁴ und/oder R⁵ gleich oder verschieden sein können und ausgewählt sind unter H, C₁-C₃-Alkyl-, C₁-C₃-Alkoxy-, Carbonsäureund Sulfonsäuregruppen.

Bevorzugte Katalysatoren der vorstehend genannten allgemeinen Formel (I) sind Phosphine, bei denen zumindest zwei der Reste R¹, R² und R³ gleich sind. Besonders bevorzugt sind Phosphine, bei denen die Substituenten R¹, R² und R³ gleich sind.

Ganz besonders bevorzugt sind substituierte Phosphine, bei denen die Reste R¹, R², R³ Phenyl-, o-Toluyl-, m-Toluyl- oder p-Toluyl-Gruppen entsprechen. Zu derartigen besonders bevorzugten Phosphinkatalysatoren gehören z.B. Tri-para-toluylphosphin, Triortho-toluylphosphin, Tris-(3-sulfophenyl)phosphin und dessen Salze, insbesondere das Trinatriumsalz, und besonders bevorzugt Triphenylphosphin.

Der Phosphinkatalysator kann auch zusammen mit einem Tri-C₁-C₁₂-alkylamin als Cokatalysator zur Anwendung kommen. Die Menge an Cokatalysator kann bis zu 35 Gew.-%, bezogen auf das Gesamtgewicht des Katalysators, betragen. Beispiele für Cokatalysatoren sind Triethylamin, Tri-n-propylamin, Tri-n-butylamin etc.

Die beschriebenen Phosphinkatalysatoren eignen sich insbesondere zur Umsetzung von Bisphenolen der Formel 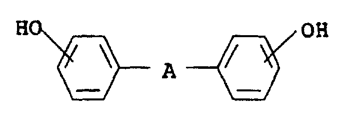 worin A für eine geradkettige oder verzweigte C₁-C₄-Alkylengruppe 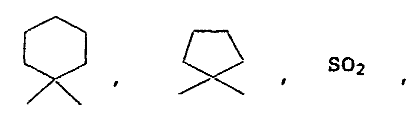 -CH₂OCH₂-, -O- oder -S- steht. Vorzugsweise steht A für

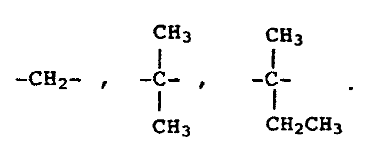

Die OH-Gruppen stehen vorzugsweise in 4- bzw. 4'-Position.

Besonders bevorzugt handelt es sich um Bisphenol A (2,2-Bis(4-hydroxyphenyl)propan), Bisphenol B (2,2-Bis(4-hydroxyphenyl)butan), Bisphenol C (1,4-Bis(4-hydroxyphenyl)cyclohexan) und Bisphenol F (2,2'-Methylendiphenol).

Man erhält somit Bisphenolalkoxylate der Formel 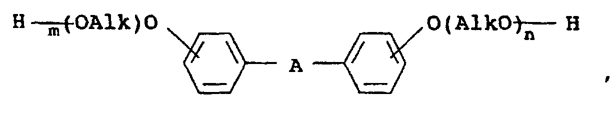 worin A die oben angegebenen Bedeutungen besitzt, m und n für 0 oder 1 stehen, wobei m und n gleich oder verschieden sein können, aber nicht gleichzeitig für 0 stehen, und Alk für eine C₂-C₄-Alkylengruppe oder für C₆H₅-CH-CH₂- und insbesondere für -CH₂-CH₂- steht.

Das erfindungsgemäße Verfahren läßt sich anhand des nachfolgenden Schemas 1 am Beispiel der Umsetzung von Bisphenol A mit Ethylenoxid erläutern.

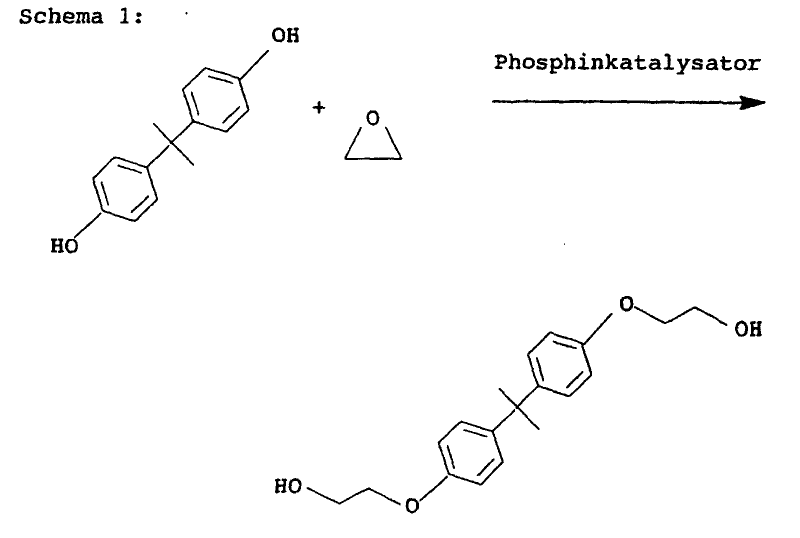

Ein ganz besonderer Vorteil des erfindungsgemäßen Verfahrens ist die hohe Reaktionsselektivität, insbesondere unter Vermeidung eines Lösungsmitteleinsatzes.

Das erfindungsgemäße Verfahren wird bevorzugt bei Temperaturen von 90°C bis 180°C durchgeführt. Der Druck liegt dabei im Allgemeinen im Bereich von 1 bis 50 bar, vorzugsweise 1 bis 20 bar, insbesondere 2 bis 15 bar.

Der Katalysator wird im Allgemeinen in einer Menge von 0,01 bis 5 Gew.-%, vorzugsweise 0,1 bis 5 Gew.-%, insbesondere 0,1 bis 2 Gew.-%, bezogen auf eingesetztes Bisphenol, verwendet. Im Allgemeinen wird die Umsetzung im Wesentlichen wasserfrei durchgeführt, d.h. der Wassergehalt des Reaktionsgemisches ist ≤ 1 Gew.-%, bezogen auf das Gewicht des Reaktionsgemisches.

Die Menge an eingesetztem Alkylenoxid richtet sich nach dem gewünschten Produkt. Im Allgemeinen verwendet man es in einer Menge von etwa 1,9 bis 2,5 Äquivalenten, bezogen auf Bisphenol. Nach beendeter Umsetzung wird das Alkylenoxid in üblicher Weise entfernt, z.B. durch Anlegen eines Vakuums.

Die entsprechend dem vorstehenden Schema 1 erhaltenen Bisphenol-Dialkoxylate werden in einer bevorzugten Ausführungsform des erfindungsgemäßen Verfahrens in einer Folgereaktion durch Umsetzung mit einem Alkylenoxid (Ethylen-, Propylen-, Butylen- oder Styroloxid) in die entsprechenden höher alkoxylierten Bisphenol-Alkoxylate überführt. Als Katalysator verwendet man dabei ein Alkalimetallhydroxid, wie NaOH, KOH, CsOH oder LiOH, ein Erdalkalimetallhydroxid, wie Magnesium- oder Calciumhydroxid oder einen DCM-Katalysator, wie z.B. in der WO 99/16775 beschrieben. Die Folgereaktion wird in dem nachfolgenden Schema 2 mit KOH als Katalysator und Ethylenoxid verdeutlicht:

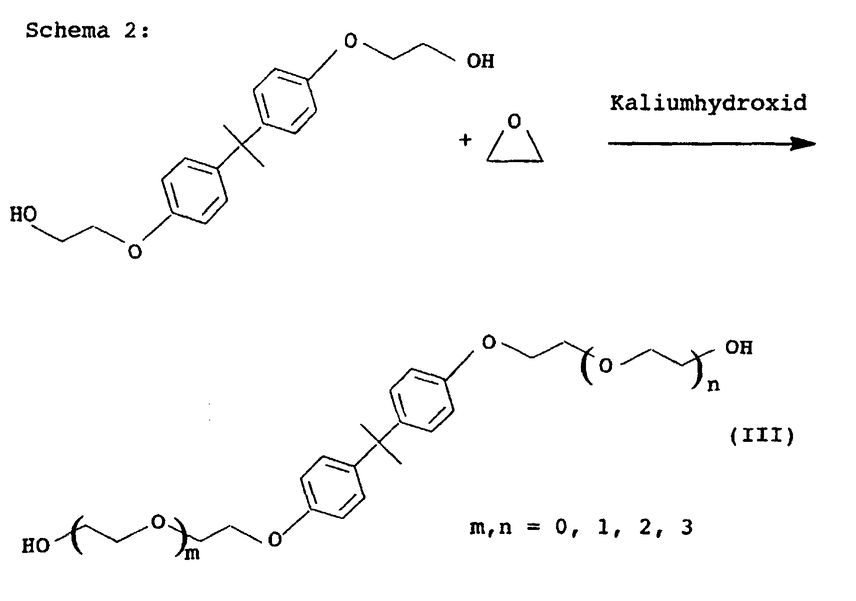

In diesem Schema können m und n verschieden oder identisch sein und für 0 bis 20, insbesondere 0, 1, 2 oder 3 stehen, wobei m und n nicht gleichzeitig für 0 stehen können. Überraschenderweise hat sich gezeigt, daß auch die höher alkoxylierten Verbindungen in reinerer Form als nach dem Stand der Technik hergestellt werden können. Bevorzugt sind die Synthesen, die zu Verbindungen mit m=n=1 führen.

Die oben beschriebene Folgereaktion wird im Wesentlichen unter den gleichen Bedingungen durchgeführt wie die Umsetzung des Bisphenols mit dem Alkylenoxid.

Nachfolgend werden beispielhaft Bisphenolethoxylate aufgeführt, die mittels des erfindungsgemäßen Verfahrens synthetisiert werden können:

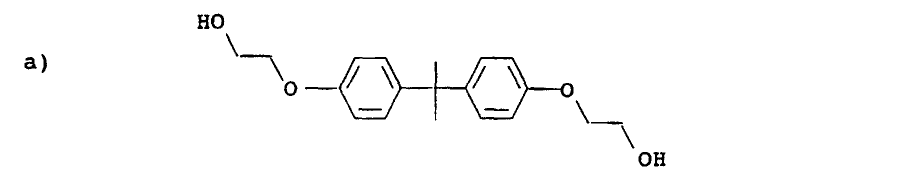

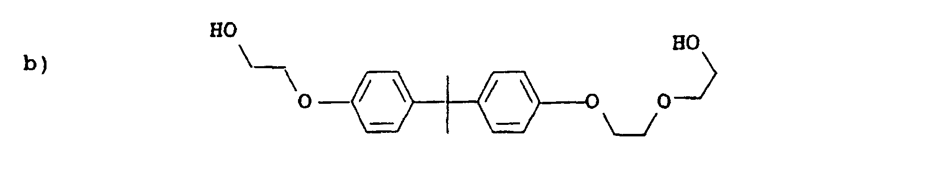

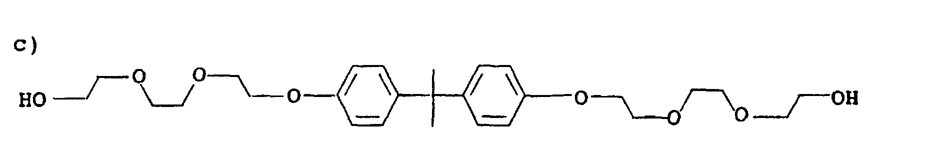

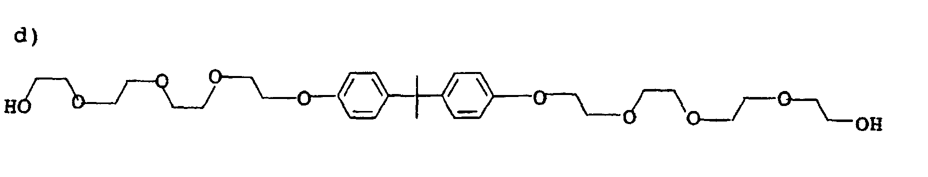

Die Erfindung wird durch das nachfolgende Beispiel näher erläutert.

### Beispiel 1

548 g Bisphenol A und 3,15 g Triphenylphosphin wurden zusammen aufgeschmolzen. Anschließend wurden 215,8 g Ethylenoxid innerhalb 2,9 h bei 120 bis 170°C zugegeben. Nach Zulaufende wurde in dem erwähnten Temperaturintervall bis zur Druckkonstanz nachgerührt. Nachdem ca. 1 bis 2 Stunden Vakuum angelegt wurde, wurde das Produkt (765 g) aus dem Reaktor abgelassen. Gemäß GC-Analyse hatte das Produkt einen Gehalt an Verbindung der obigen Formel a) von 92,7%.

## Patentansprüche

1. Verfahren zur Herstellung von Bisphenolalkoxylaten, wobei man mindestens ein Bisphenol mit einem Alkylenoxid in Anwesenheit eines im Wesentlichen alkalimetallhydroxidfreien Phosphinkatalysators zur Reaktion bringt.

2. Verfahren nach Anspruch 1, wobei die Reaktion in der Schmelze erfolgt.

3. Verfahren nach Anspruch 1 oder 2, wobei der Phosphinkatalysator ausgewählt ist aus der Gruppe der substituierten Phoshine der nachfolgenden Formel (I): 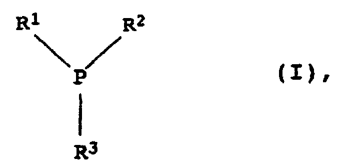 worin R¹, R² und R³ Arylreste der nachfolgenden Formel (II) sind, 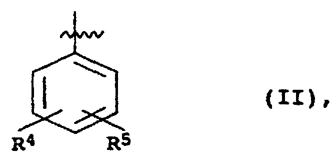 worin R⁴ und R⁵ gleich oder verschieden und ausgewählt sind aus der Gruppe der Reste, bestehend aus H, C₁-C₃-Alkyl-, C₁-C₃-Alkoxy-, -COOM und SO₃M-Gruppen, wobei M für H oder ein Alkalimetall steht.

4. Verfahren nach Anspruch 3, wobei Phosphinkatalysatoren gemäß Formel (I) eingesetzt werden, bei denen die Reste R¹, R² und R³ identisch sind.

5. Verfahren nach Anspruch 3 oder 4, wobei es sich bei den Resten R¹, R² oder R³ um Phenyl-, o-Toluyl-, m-Toluyl-, p-Toluyl-, m-Sulfophenyl und/oder Natrium-m-sulfophenyl handelt.

6. Verfahren nach Anspruch 5, wobei man als Katalysator Triphenylphosphin, Tri-o-toluylphosphin oder Tris-(3-sulfophenyl)phosphin oder ein Salz davon verwendet.

7. Verfahren nach einem der vorhergehenden Ansprüche, wobei man ein Bisphenol der Formel 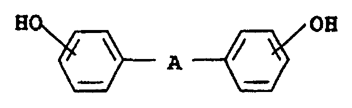 worin A für eine geradkettige oder verzweigte C₁-C₄-Alkylengruppe, 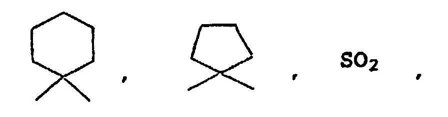 -CH₂OCH₂-, -O- oder -S- steht, verwendet.

8. Verfahren nach einem der vorhergehenden Ansprüche, wobei man zunächst ein Bisphenoldi-monoalkoxylat herstellt und dieses dann zu dem Mehrfachalkoxylat der nachfolgenden Formel (III) umsetzt 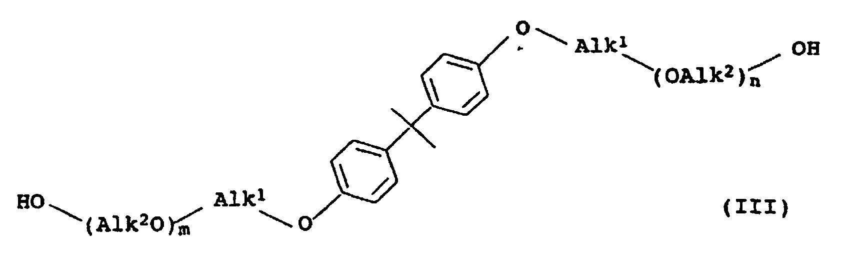 wobei m und n, gleich oder verschieden sind und für 0, 1, 2 oder 3 stehen, wobei m und n nicht gleichzeitig für 0 stehen können, und Alk¹ und Alk², die gleich oder verschieden sein können, für C₂-C₄-Alkylen oder 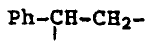 oder Kombinationen davon stehen.

9. Verfahren nach einem der vorhergehenden Ansprüche, wobei man als Alkylenoxid Ethylenoxid verwendet.

## Claims

1. A process for preparing bisphenol alkoxylates, comprising reacting at least one bisphenol with an alkylene oxide in the presence of a phosphine catalyst which is essentially free of alkali metal hydroxides.

2. A process as claimed in claim 1, wherein the reaction is carried out in the melt.

3. A process as claimed in claim 1 or 2, wherein the phosphine catalyst is selected from the group consisting of substituted phosphines of the formula (I) : 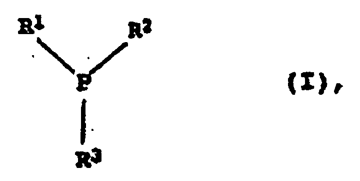 where R¹, R² and R³ are aryl radicals of the formula (II), 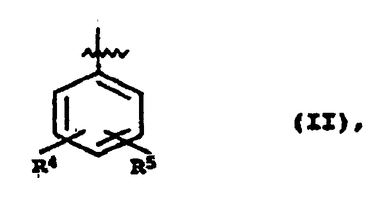 where R⁴ and R⁵ are identical or different and are selected from the group consisting of H, C₁-C₃-alkyl groups, C₁-C₃-alkoxy groups, -LOOM and SO₃M groups, where M is H or an alkali metal.

4. A process as claimed in claim 3, wherein phosphine catalysts of the formula (I) in which the radicals R¹, R² and R³ are identical are used.

5. A process as claimed in claim 3 or 4, wherein the radicals R¹, R² and R³ are phenyl, o-tolyl, m-tolyl, p-tolyl, m-sulfophenyl and/or sodium m-sulfophenyl.

6. A process as claimed in claim 5, wherein the catalyst used is triphenyl phosphine, tri-o-tolylphosphine or tris(3-sulfophenyl)phosphine or a salt thereof.

7. A process as claimed in any of the preceding claims, wherein a bisphenol of the formula 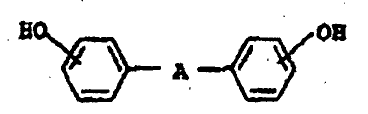 where A is a straight-chain or branched C₁-C₄-alkylene group, 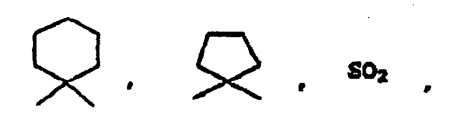 -CH₂OCH₂-, -O- or -S-, is used.

8. A process as claimed in any of the preceding claims, wherein a bisphenol dimonoalkoxylate is prepared first and this is then reacted to give the polyakoxylate of the formula (III) 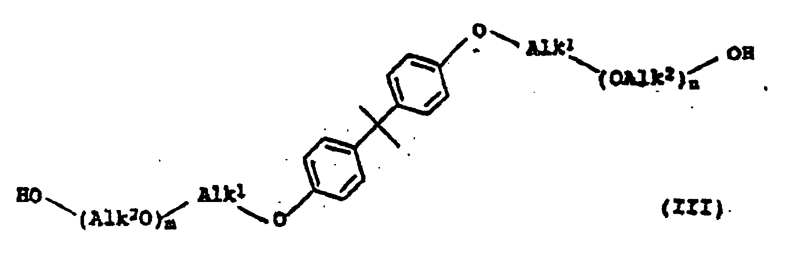 where m and n are identical or different and are each 0, 1, 2 or 3, with m and n not simultaneously being able to be 0, and Alk¹ and Alk² can be identical or different and are C₂-C₄-alkylene or 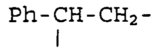 or combinations thereof.

9. A process as claimed in any of the preceding claims, wherein the alkylene oxide used is ethylene oxide.

## Revendications

1. Procédé pour la préparation d'alcoxylates de bisphénol, dans lequel fait réagir au moins un bisphénol avec un oxyde d'alkylène en présence d'un catalyseur de phosphine essentiellement exempt d'hydroxyde de métal alcalin.

2. Procédé selon la revendication 1, dans lequel on réalise la réaction dans la masse fondue.

3. Procédé selon la revendication 1 ou 2, dans lequel on choisit le catalyseur de phosphine dans le groupe formé par les phosphines substituées de formule (I) suivante : 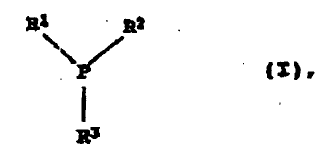 dans laquelle R¹, R² et R³ représentent des groupes aryle de formule (II) suivante : 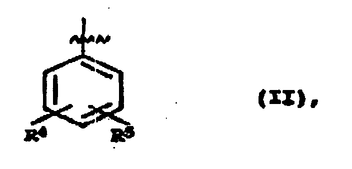 dans laquelle R⁴ et R⁵ sont identiques ou différents et sont choisis dans le groupe formé par un atome d'hydrogène et les groupes alkyle en C₁ à C₃, alcoxy en C₁ à C₃, -COOM et SO₃M, dans lesquels M représente un atome d'hydrogène ou un atome de métal alcalin.

4. Procédé selon la revendication 3, dans lequel on met en oeuvre des catalyseurs de phosphine selon la formule (I) dans lesquels les groupes R¹, R² et R³ sont identiques.

5. Procédé selon la revendication 3 ou 4, dans lequel les groupes R¹, R² ou R³ représentent un groupe phényle, o-toluyle/ m-toluyle, p-toluyle, m-sulfophényle et/ou m-sulfophényle sodique.

6. Procédé selon la revendication 5, dans lequel on met en oeuvre, en tant que catalyseur, la triphénylphosphine, la tri-o-toluylphosphine ou la tris-(3-sulfophényl)phosphine ou un de leurs sels.

7. Procédé selon l'une quelconque des revendications précédentes, dans lequel on met en oeuvre un bisphénol de formule 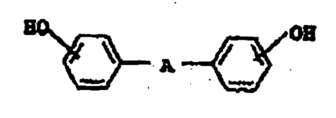 dans laquelle A représente un groupe alkylène en C₁ à C₄ à chaîne linéaire ou ramifiée, un groupe 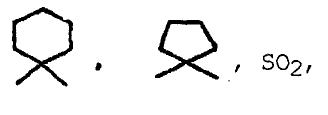 -CH₂OCH₂-, -O- ou -S-.

8. Procédé selon l'une quelconque des revendications précédentes, dans lequel on prépare, dans un premier temps, un di-monoalcoxylate de bisphénol que l'on fait ensuite réagir pour obtenir un alcoxylate multiple de formule (III) suivante : 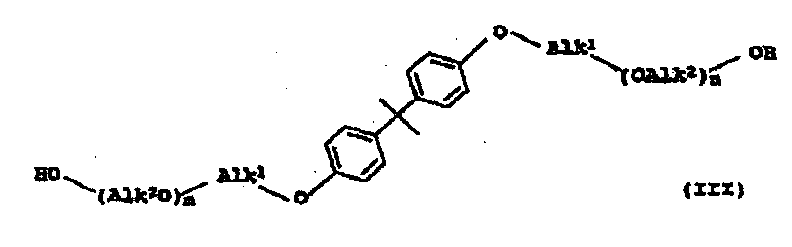 dans laquelle m et n sont identiques ou différents et valent 0, 1, 2 ou 3, m et n ne pouvant pas valoir en même temps 0, et dans laquelle Alk¹ et Alk², pouvant être identiques ou différents, représentent un groupe alkylène en C₂ à C₄ ou 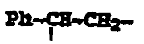 ou une combinaison de ceux-ci.

9. Procédé selon l'une quelconque des revendications précédentes, dans lequel on met en oeuvre, en tant qu'oxyde d'alkylène, l'oxyde d'éthylène.
